Europäisches Patentamt

European Patent Office

Office européen des brevets

(11) Publication number: **0 188 252 B1**

(12) **EUROPEAN PATENT SPECIFICATION**

(45) Date of publication of patent specification: **02.09.92**

(51) Int. Cl.⁵: **C12P 13/02**, C12N 13/00, //C12N9/78

(21) Application number: **86100307.7**

(22) Date of filing: **11.01.86**

(54) **Process for producing amides by use of microorganisms.**

(30) Priority: **11.01.85 JP 2724/85**

(43) Date of publication of application:
**23.07.86 Bulletin 86/30**

(45) Publication of the grant of the patent:
**02.09.92 Bulletin 92/36**

(84) Designated Contracting States:
**BE DE FR GB IT NL**

(56) References cited:
GB-A- 2 018 240
US-A- 3 880 717

CHEMICAL ABSTRACTS, vol. 103, no. 1, July 1985, page 465, abstract no. 5046z, Columbus, Ohio, US; & JP-A-59 213 387 (NIPPON CARBIDE INDUSTRIES CO., INC.) 03-12-1984

(73) Proprietor: NITTO KAGAKU KOGYO KABUSHIKI KAISHA
5-1, Marunouchi 1-chome
Chiyoda-Ku Tokyo-To(JP)

Proprietor: MITSUBISHI RAYON CO., LTD.
3-19, Kyobashi 2-chome Chuo-Ku

Tokyo 104(JP)

(72) Inventor: **Enomoto, Kanehiko**
36-5-106, Kyodo 5-Chome Setagaya-Ku Tokyo-To(JP)
Inventor: **Sato, Yoshiaki**
138-21, Setogaya-Cho Hodogaya-Ku Yokohama-Shi Kanagawa-Ken(JP)
Inventor: **Nakashima, Yasutaka**
2-6-206, Kurokawa 3-Chome
Otake-Shi Hiroshima-Ken(JP)
Inventor: **Fujiwara, Atsushi**
245-1, Kamihoshikawa Hodogaya-Ku Yokohama-Shi Kanagawa-Ken(JP)
Inventor: **Doi, Toshiaki**
298-8, Setogaya-Cho Hodogaya-Ku Yokohama-Shi Kanagawa-Ken(JP)

(74) Representative: **Reichel, Wolfgang, Dipl.-Ing. et al**
Reichel und Reichel Parkstrasse 13
W-6000 Frankfurt am Main 1(DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

BACKGROUND OF THE INVENTION

Field of the Art

This invention relates to a process for hydrating a nitrile compound by the action of nitrilase produced by microorganisms to convert the nitrile compound into the corresponding amide compound. More particularly, this invention relates to the process for conducting the hydration reaction which does not substantially proceed without irradiation with light by irradiation with light of the nitrilase.

Recently there have been developed processes for carrying out chemical reactions by the use of microorganisms or enzymes obtained therefrom. In general, the reactions by means of microorganisms or enzymes are advantageous in that consumption of energy for the reactions is small because the reaction can be carried out at room temperature and atmospheric pressure and that the desired products of high purity are easily obtained because the selectivity of the reaction to yield the desired products is very high. On the other hand, there is room for improvement with respect to the reaction activity, the life time of microorganisms or enzymes used as catalysts. Especially, there are problems when the velocity of the desired reaction (i.e. reaction activity) is low under its optimum conditions and especially at its optimum temperature and/or pH. In such a case, the productivity per bacterial cells used is reduced because large capacity reactors are required owing to low space yield, reaction takes a longer time owing to a slow reaction velocity, and also the reaction activity is lowered.

Thus, it is fundamentally important to realize a high reaction activity of the microorganisms or enzymes to be used. Such reaction activity is a main economical factor of industrial production.

Prior Art

Processes for hydrating nitrile compounds to produce the corresponding amide compounds by the action of the nitrilase produced by microorganisms having enzymatic activity which hydrates a nitrile compound into the corresponding amide compound (i.e. nitrilase activity) are described in Japanese Patent Publication No.17918/81 and No.38118/81 and Japanese Laid-Open Patent Application (Kokai)) No.86186/76 Specifications. Microorganisms play an important role in these reactions, and several microorganisms are disclosed in the specifications referred to above.

Problems

It has been found by the present inventors that the nitrilase activity cannot be exhibited sufficiently when a large metal reaction apparatus is used in order to carry out hydration reaction of nitrile compounds in an industrial scale by utilizing the nitrilase produced by these microorganisms. It has been uncertain whether these phenomena are caused by the microorganisms themselves or the material and structure of the reaction apparatus or other reasons. Thus it was necessary to solve these problems for industrial operation of a microbiological process for producing amide compounds.

A solution

As a solution for the above problems, the present inventors have already made an invention (Japanese Patent Application No. 125588/83), which has the feature that the microorganism to be used is allowed to accept light energy over a certain amount in carrying out the hydration reaction in an reaction vessel composed at least partly of a non-light-transmitting material. This invention is based on the findings that (i) the microbial cells exhibit no nitrilase activity without irradiation with light, (ii) the nitrilase activity realized by irradiation with light decreases with time, (iii) an increase in the nitrilase activity (including the increase from 0) by irradiation with light is observed.

At the time the invention was made, it was recognized by the inventors that the effect of the light irradiation according to the invention owes to enhancement of the velocity of substrate (nitrile) and product (amide) permeating through cell membranes. In fact, the increase in the nitrilase activity by irradiation with light was not observed when the nitrilase activity of a specimen enzyme which has no connection with the permeation through cell membranes was utilized. Later research after the invention, however, has made it clear that the nitrilase activity of a specimen enzyme is also realized for the first time when it is irradiated with light, and that the nitrilase activity once realized hardly decreases with time. This accounts for the reason why the increase in the nitrilase activity of the above specimen enzyme was not observed. The nitrilase activity of the specimen enzyme used in the test had already been realized by irradiation with light in the preparation of the specimen enzyme and had been maintained without decrease thereof when the test was made, and thus there was no room for a further increase in the nitrilase activity by irradiation with light in the test.

SUMMARY OF THE INVENTION

The present invention is based on the finding described above.

According to the present invention, there is presented a process for hydrating a nitrile compound by the action of nitrilase produced by a microorganism to convert the nitrile compound into the corresponding amide compound, characterized by conducting the reaction under the conditions wherein:

(a) the nitrilase produced by the mircroorganism is in the form of crushed cells of the microorganism or intra-cellular aubstance obtained from the microorganism;

(b) the nitrilase produced by the microorganism is allowed to accept light energy of light having a wavelength of 100 to 1000 nm corresponding to at least $10\mu$E/g microbial cells ($\mu$mol of photons/g microbial cells) before termination of the hydration reaction; wherein it is excluded that the accepted light energy is supplied solely by natural daylight; and

(c) the hydration reaction is carried out in a vessel composed at least partly of a material which does not permit transmission of light therethrough.

According to the present invention are given, in the first place, the advantages inherent in the above-mentioned prior invention by the present inventors. That is, in the hydration reaction of a nitrile compound by the action of nitrilase produced by a microorganism according to the present invention, (i) the hydration reaction which does not substantially proceed in a reaction vessel composed of a non-light-transmitting material where light is substantially absent can proceed by irradiation of the nitrilase with light, and (ii) the hydration reaction which proceeds to some extent in a reaction vessel composed partly of a non-light-transmitting material where some amount of light is present can markedly proceed by the irradiation with light.

Further, the present invention has its inherent advantage that the hydration reaction can be carried out at a completely dark place thanks to the maintenance of nitrilase activity once realized by irradiation with light of the crushed cells or the intra-cellular substance of a microorganism. In view of the fact that reaction apparatuses for industrial use are made of metal, it is advantageous for industrial operation of the hydration reaction that no device for light irradiation is required to be taken on such apparatuses.

It is well known that useful substances are produced according to microbiological methods including the use of enzymes. However, realization or enhancement of the activity of microorganisms or their enzymes by irradiation with electromagnetic waves such as light is not believed to have been well known. On the contrary, it is generally believed that the irradiation with light is often harmful. Thus, it can be said that the effect of irradiation with light which is exhibited only in the above mentioned case has not been expected in view of the usual results of light on microorganisms or their enzymes.

DETAILED DESCRIPTION OF THE INVENTION

Nitrilase produced by a microorganism

As described above, it is known that a nitrile compound is converted to the corresponding amide compound by the action of microorganisms having nitrilase activity or the nitrilase produced by the microorganisms. The present inventors have found that the microorganisms belonging to the genuses described in the above mentioned Japanese Patent Publication Nos. 17918/81 and 38118/81 and Japanese Laid-Open Patent Application No.86186/76 Specifications generally have improved activity by the irradiation thereof with light and the crushed cells of the microorganisms and the intra-cellular substance taken out of the cells can be used in the present invention.

Specific examples of such microorganisms include the bacteria belonging to the following genuses:
(a) Corynebacterium;
(b) Nocardia;
(c) Bacillus;
(d) Bacteridium;
(e) Micrococcus; and
(f) Brevibacterium.

3

Specific strains belonging to these genuses include: Corynebacterium --- N-771 strain (FERM P 4445 deposited at a Japanese depository, Fermentation Research Institute, Agency of Industrial Science and Technology, 1, 3-Higashi 1-chome, Yatabe-machi, Tsukuba-gun, Ibarakiken, Japan, which depository is herein called FRI, on May 30, 1978) and N-774 strain (FERM P 4446 deposited at FRI on May 30, 1978); Nocardia genus --- N-775 strain (FERM P 4447 deposited at FRI on May 30, 1978), these strains being described in Japanese Patent Publication No. 17918/81 and No.38118/81 Specifications; and the strains of Bacillus genus and others which are described in Japanese Laid-Open Patent Application No.86186/1976 Specification and reported to have been deposited at CBS*) and FRI. The bacteriological properties of these strains are described in these publicly known specifications.

The nitrilase activity utilized in the present invention is that of the crushed cells of the above microorganisms or that of the intra-cellular substance obtained from the microorganisms. The "crushed cells" herein means (i) cells crushed by French Press or other mechanical means, (ii) cells which are burst by osmotic pressure in a liquid medium wherein the cell membranes and cell walls are disrupted, and (iii) others, the nitrilase or the intra-cellular substance having nitrilase activity existing outside the cells and the residue of the cell membranes and cell walls being not yet removed. When the term "the intra-cellular substance obtained from the microorganisms" is used, the "intra-cellular substance" means at least a part of the whole substance, in concrete terms, (i) the above mentioned crushed cells wherein the cell membranes and cell walls have been removed, (a part of the intra-cellular substance could also be removed in the removal as described above), (ii) specimen enzymes obtained from such substance as described in the above (i) by conventional methods such as ammonium sulfate fraction (Needless to say, they can have a variety of nitrilase content or titer), and (iii) others.

For the sake of simplicity, all of these are called hereinafter in a general name "the crushed cells, etc.".

Nitrile compounds and amide compounds

The nitrile compounds are represented by the general formula $R-(CN)_n$ and encompass broad ranges of compounds. The compounds include mononitriles when $n = 1$ and polynitriles when $n \geq 2$. The R is hydrogen or a saturated or unsaturated hydrocarbon residue which has a variety of numbers of carbon atoms and also has a straight, branched or cyclic chain. The hydrocarbon residue can further contain amino group, hydroxyl group, a halogen, carboxyl group or other substituents which are compatible with the microorganism used.

Examples of preferable nitrile compounds include acetonitrile, propionitrile, n-butyronitrile, isobutyronitrile, n-valeronitrile, acrylonitrile, methacrylonitrile, benzonitrile, cyanopyridine, malononitrile, succinonitrile, fumaronitrile, chloroacetonitrile, $\beta$-hydroxypropionitrile, aminoacetonitrile and $\beta$-aminopropionitrile.

As a result of experiments with many nitrile compounds as shown in the following Examples, it has been found that the nitrilase activity is enhanced without exception by the irradiation with light. Thus, it is said that the present invention can be generally established with respect to nitrile compounds.

The resulting hydration reaction product is the corresponding amide compound wherein the CN group of the starting nitrile compound was hydrated into a $CONH_2$ group.

From the viewpoint of usefulness of the products, important at least at present will be the production of acrylamide from acrylonitrile and nicotinic acid amide from cyanopyridine.

Hydration reaction

It is known to carry out the hydration reaction of a nitrile compound under the action of nitrilase produced by microorganisms having nitrilase activity. In the present invention, the hydration can be conducted in any desired embodiment so far as the object of the invention is not impaired. It is to be noted that no alteration in the hydration reaction itself has been observed by irradiation with light.

The method of hydration reaction generally comprises contacting the starting nitrile compound with the crushed cells, etc. in an aqueous medium for a predetermined period of time.

The crushed cells, etc. can be in the form of crushed cells in a liquid culture; crushed cells separated from the culture medium; a dried product of the crushed cells; or recovered specimen enzymes or those supported on or immobilized in a liquid or solid carrier. The preferred form of the crushed cells, etc. are those immobilized in a polymer gel such as crosslinked polyacrylamide gel or crosslinked polyvinyl alcohol.

The concentrations of the substrate (nitrile) and the crushed cells, etc. in the aqueous medium can be suitably selected, and are generally in a concentration of the substrate ranging from about 0.01 to about 5% by weight and in a concentration of the crushed cells, etc. ranging from about 0.01 to about 2% by weight.

*) CBS=Centraal Bureau Voor Schimmelstructuren, Baarn, The Netherlands

The concentration of the crushed cells, etc. is herein expressed on the basis of a dry weight of the original cells. An appropriate conversion is thus necessary for the use of the specimen enzymes as the crushed cells, etc. The reaction temperature, reaction pH and other reaction conditions can be determined, depending on the type of the crushed cells, etc. to be used. Normally, the reaction temperature is in the range of about 0 to about 20°C, and the pH is in the vicinity of about 7. A suitable buffer agent can be used in order to maintain the pH at a predetermined level.

Irradiation with light

(1) Light

The irradiation with light is conducted onto the crushed cells, etc. which are in the stage before termination of the hydration reaction. The term "before termination of hydration reaction" means any point of time before termination of the hydration reaction, wherein the termination does not always mean the conversion of 100%. Since the irradiation with light is conducted onto the crushed cells, etc., the stage before termination of hydration reaction also includes the point of time before contacting the crushed cells, etc. with a nitrile compound. Accordingly, the irradiation can be conducted before and/or after contacting the crushed cells, etc. with a nitrile compound.

The "light" to be irradiated according to the present invention can have any wavelength as far as the effect of irradiation is exhibited. However, as readily understood in view of the light being a region of electromagnetic waves, the effect is small when the light is in a longer wavelength region emitted from a lower energy atomic level. On the other hand, when the wavelength is too short, its energy is too large and the molecules (arrangement) of enzymes will be destroyed. Thus, the crushed cells, etc. will lose their enzymatic activity.

In accordance with the inventors' research in the broad range of wavelength regions, the light having a long wavelength over 800 nm has no abnormal effect on the crushed cells, etc., but its effect of enhancing the enzymatic activity is not remarkable. When the light has a short wavelength less than 100 nm, the effect of enhancing the activity can be exhibited in a short period of time, but the crushed cells, etc. irradiated result in unrecoverable decrease in their activity. Thus, the light preferred in the present invention has a wavelength of 100 to 1,000 nm. Specifically, such light can be obtained, for example, from a light for sterilization in a wavelength range of 200 to 300 nm, a blacklight lamp in a wavelength range of 300 to 400 nm, and a light for general irradiation in a wavelength range of 400 to 800 nm.

The irradiation with light can be carried out with any strength or amount as far as the effect of enhancing the activity is observed. More specifically, the irradiation should be conducted so that the crushed cells, etc. receive light energy of at least about 10 $\mu$E/g cells. A preferred amount of light energy varies with the wavelength of light to be irradiated. Specifically, the irradiation should be conducted so that the crushed cells, etc. receive light energy of at least about 10 $\mu$E/g cells, preferably at least about 20 $\mu$E/g cells when a light having wavelength of 300 to 450 nm is used, and at least about 50 $\mu$E/g cells, preferably at least about 100 $\mu$E/g cells when a light scarcely having wavelength of 300 to 450 nm is used. Incidentally, the use of immobilized crushed cells, etc. sometimes decreases the reaction velocity by from about 1/2 to about 1/3 in comparison with the use of non-immobilized crushed cells, etc. at the same concentration. In this case, the irradiation requires light energy about 2 to 3 times as large as the above mentioned value. As to the light energy, the unit "E" is the amount of light energy which corresponds to the energy of 1 mole of photons (h$\nu$), i.e. the energy depends on the frequency ($\nu$) of the irradiating light. The unit "g cells" is a dry weight expressed in gram of the original cells of the crushed cells, etc., and therefore an appropriate conversion is necessary for the use of specimen enzymes. That is the reason why the expression "light energy corresponding to" is used in claim 1. The "second" as used further below is the period of irradiation.

The amount of light energy received by the crushed cells is larger than that of the environmental light present in a reaction vessel or other reaction apparatus of an ordinary industrial scale, i.e., larger than that which is given by room light and/or scattered sunlight into a room. More particularly, according to the inventors' experiment, the luminous intensity at industrial production sites is normally about 100 lx. At such intensity of light, the amount of light energy received by a reaction vessel as large as about 250 liters of capacity will be short of the above defined 10 $\mu$E/g cells, even when the light is irradiated for several hours and the concentration of the crushed cells, etc. is as low as 1,000 ppm and the upper portion of the reaction vessel is fully exposed to light.

Thus, in industrial production sites, it is difficult to satisfy the required amount of light energy even under the conditions accessible to light. When the irradiation with light is conducted according to the present invention, the room light if any given to the crushed cells, etc. is included in the total amount of the light energy of at least about 10 $\mu$E/g cells.

In order to obtain the required reaction velocity in the process of the present invention, the irradiation is carried out, for example, at the lowest light energy of 10 $\mu$E/g cells.

(2) Irradiation

The irradiation can be conducted in any way, as far as the crushed cells, etc. before or in contact with a nitrile compound can receive the required amount of light energy from a light source for irradiation.

The term "a light source for irradiation of the crushed cells, etc." means a light source placed inside or outside of the reaction apparatus for irradiation of the crushed cells, etc. with light, and also includes some light such as scattered sunlight and/or room light which may come into the reaction apparatus surrounding the reaction apparatus. Incidentally, when one makes the room where a reaction apparatus is installed excessively or extraordinarily bright so that significant light energy from the room light can come into the reaction apparatus, such a light source should be understood as a light source for irradiation according to the present invention.

More specifically, the irradiation with light can be conducted by, for example, (a) a light source placed at an upper space over a vessel containing the crushed cells, etc. (i.e. space over the crushed cells, etc. or over an aqueous suspension thereof), (b) irradiation through a window equipped at a top, side or bottom portion of the reaction vessel with an outside light source, (c) irradiation by means of a lamp placed within a liquor in the reaction vessel so that the vessel can also be used as a photochemical reactor, and (d) other methods. As necessary and if possible, reaction liquor is taken out of a vessel (especially a reaction vessel) containing the crushed cells, etc., and the liquor is then irradiated in another vessel by a method as described above. The vessel for irradiation in this case can, for example, comprise one or more transparent glass tubes.

The effect of light irradiation according to the present invention can be markedly exhibited, when a large reservoir of the crushed cells, etc. or reaction vessel of industrial scale is used wherein the incident light energy from an ordinary room light is short of the required amount of light energy. Such a large reservoir or reaction vessel is normally made of a non-light-transmitting material and especially of a metal material in its substantial portions. In such a non-light-transmitting reservoir or reaction vessel, the light energy obtained from ordinary illumination through a window or a cover of the vessel is very small. Thus, positive irradiation with light is indispensable. Incidentally, the term "reaction vessel" herein means a portion of reaction apparatus wherein at least major hydration reaction is carried out.

Differently from the case where nitrilase activity is brought about by the use of microorganisms, irradiation with light once conducted is enough in the case where the crushed cells, etc. are used as the source of nitrilase, which is a significant feature of such a source of nitrilase. Therefore, in a preferable embodiment of the present invention which makes the most of such a feature, irradiation with light in a sufficient amount is conducted before or in crushing cells or in the treatment of crushed cells (including the preparation of specimen enzymes), and the hydration reaction itself is conducted without a care for the irradiation.

Experimentation

Example 1

(1) Culture of cells

A medium (pH 7.2) comprising glucose 1%, peptone 0.5%, yeast extract 0.3%, malt extract 0.3%, and ferrous sulfate•7 hydrate 0.05% was placed into a 500 ml Erlenmeyer flask. After sterilization, a loopful of N-774 strain (Corynebacterium) was inoculated into the medium and was cultured at 30°C for 2 days.

(2) Preparation of dark standing cells and light standing cells

According to a conventional method, the cultured cells were gathered by centrifugalization and were washed with a 0.05M phosphate buffer (pH 7.7) to obtain washed cells. The cells were dispersed in a 0.05M phosphate buffer to obtain a suspension of the cells having a concentration of 17.3 g dried cells/liter.

Dark standing cells: The cell suspension was allowed to stand at 0°C at a dark place for 3 to 4 days to prepare dark standing cells.

Light standing cells: The dark standing cells were allowed to stand at 0°C for 1 hour under irradiation at light energy of 5.7 $\mu$E/g cells•second using a daylight lamp (Model DR 400/T, supplied by Tokyo Shibaura Denki K.K., Japan, wavelength: about 300 to about 800 nm) to prepare light standing cells.

(3) Preparation of crushed cells and intra-cellular substance

Crushed cells: 100 ml of a suspension of the dark standing (or light standing) cells was pressured three times by French Press (supplied by Otake Seisakusho K.K., Japan) at a pressure of 1300 kg/cm$^2$ at a dark place to crush the cells, and about 90 ml of a solution containing the crushed cells was obtained, hereinafter this solution being referred to as "crushed cell solution".

Intra-cellular substance: About 50 ml of the crushed cell solution was centrifuged at 15,000 rpm using a high-speed centrifuge to obtain about 45 ml of a liquid extract from which the residual cells have been removed. In accordance with a conventional method, the liquid extract was subjected to removal of nucleic acid and ammonium sulfate fractionation to obtain about 15 ml of a crude enzyme solution disposed with dialysis belonging to a fraction given by 50 to 60% saturation of ammonium sulfate.

Incidentally, all of the above operations were conducted quickly in a low-temperature room wherein the temperature was 4°C and the intensity of light irradiation was 0.02 $\mu$E/m$^2$•second or less, unless otherwise specified.

(4) Measurement of reaction velocity

0.2 ml of cell suspension or crushed cell solution or liquid extract or crude enzyme solution and 4.8 ml of 0.05M phosphate buffer (pH 7.7) were mixed, and then 5 ml of 0.05M phosphate buffer (pH 7.7) containing 5% by weight of acrylonitrile was added thereto. The mixture was subjected to reaction at 0°C for a predetermined period of time. After the termination of the reaction, acrylamide produced was subjected to quantitative analysis by gas chromatography to measure a reaction velocity. In the reaction, non-light-transmitting reaction tubes wound with a black tape were used.

The velocity of acrylamide (hereinafter referred to as AA) production is shown by the amount of AA produced per 10 minutes. Comparative data of the velocity for each of the nitrilase sources as described above is shown in Table 1.

Table 1

| Kind of cells | Substance used in measurement of activity | Amount of AA produced per 10 minutes (%) |
|---|---|---|
| Dark standing cells | Cells* | 0.017 |
| | Crushed cell solution | 0.017 |
| | Liquid extract | 0.011 |
| | Crude enzyme solution | 0.015 |
| Light-standing cells | Cells* | 0.863 |
| | Crushed cell solution | 0.871 |
| | Liquid extract | 0.687 |

*Reference example

Example 2

Dark standing cells, crushed dark standing cell solution, liquid extract and crude enzyme solution both obtained from said cells were prepared as in Example 1. Each of them was irradiated with light for 10 minutes using the daylight lamp used in Example 1, and the amount of AA produced per 10 minutes was measured. Comparison of the above amount with that obtained when the above substances not irradiated were used in the reaction gives the results shown in Table 2.

Table 2

| Substance used in measurement of activity | Amount of AA produced per 10 minutes(%) | |
|---|---|---|
| | Before irradiation | After irradiation |
| Cells* | 0.017 | 0.863 |
| Crushed cells solution | 0.017 | 0.888 |
| Liquid extract | 0.011 | 0.643 |
| Crude enzyme solution | 0.015 | 0.901 |

*Reference example

## Example 3

Each of the light-irradiated cells, liquid extract and crude enzyme solution prepared in Example 2 was allowed to stand at 0°C at a dark place, and then the ratio of residual activity was measured. The results are shown in Table 3, wherein the amount of AA produced per 10 minutes which was obtained when the above substances were used immediately after the irradiation is shown by 100% and the ratio of residual activity is shown by the relative value.

Table 3

| Substance allowed to stand at a dark place | Ratio of residual activity (%) | | | |
|---|---|---|---|---|
| | Dark standing period (day) | | | |
| | 0 | 1 | 2 | 3 |
| Cells | 100 | 12.3 | 6.0 | 3.2 |
| Liquid extract | 100 | 101 | 101 | 93.3 |
| Crude enzyme solution | 100 | 107 | 98.3 | 95.7 |

## Example 4

5 ml each of liquid extract from dark standing cells prepared as in Example 1 was taken into 50 ml steel vessels and was irradiated with light having wavelength of 300 to 400 nm for 3 minutes using a blacklight lamp (Model FL-4BL-G, supplied by Tokyo Shibaura Denki K.K., Japan), wherein the light energy was controlled by the use of filters having different optical area placed at an upper portion of the vessel. As a result, several samples of liquid extract which have been accepted different total amounts of light energy were prepared and a reaction velocity was measured as in Example 1.

The influence of the total amount of light energy on the reaction velocity is expressed by a ratio to 1 which is a relative value expressing the reaction velocity in the case where liquid extract applied no light energy was used, which is shown in Table 4.

Table 4

| Total light energy applied ($\mu$E/g cells) | Ratio of reaction velocity |
|---|---|
| 0 | 1.0 |
| 3 | 2.8 |
| 10 | 8.2 |
| 30 | 15.6 |
| 60 | 23.4 |
| 180 | 44.5 |

Example 5

5 ml each of liquid extract from dark standing cells prepared as in Example 1 was taken into 50 ml steel vessels, and was irradiated with light using the daylight lamp used in Example 1, wherein the portion of light in a wavelength up to about 430 nm was removed by the use of colored glass filters (Model Y45, supplied by Tokyo Shibaura Denki K.K., Japan) each having the same optical area placed at an upper portion of the vessel and the irradiation time was different for each sample. A reaction velocity was measured as in Example 1 for each of the samples which have been accepted different total amounts of light energy.

The influence of the total amount of light energy on the reaction velocity is expressed by a ratio to 1 which is a relative value expressing the reaction velocity in the case where liquid extract applied no light energy was used, which is shown in Table 5.

Table 5

| Total light energy applied ($\mu$E/g cells) | Ratio of reaction velocity |
|---|---|
| 0 | 1.0 |
| 15 | 2.9 |
| 50 | 6.8 |
| 70 | 11.6 |
| 170 | 19.5 |
| 470 | 34.8 |

Example 6

Liquid extracts prepared as in Example 1 from the dark standing cells of various genuses, namely, Bacillus (CBS-494), Bacteridium (CBS-496), Micrococcus (CBS-497), Brevibacterium (CBS-717), and Nocardia (N-775) were irradiated with light as in Example 2. A reaction velocity was measured for the above extracts both irradiated and not irradiated.

The reaction velocity for the extract irradiated with light is expressed as a relative value to 1 which expresses the velocity for the extract not irradiated, which is shown in Table 6.

Table 6

| Genuses of cells | Strains | Reaction velocity for light-irradiated extract |
|---|---|---|
| Bacillus | CBS-494 | 23.5 |
| Bacteridium | CBS-496 | 19.8 |
| Micrococcus | CBS-497 | 20.1 |
| Brevibacterium | CBS-717 | 47.0 |
| Nocardia | N-775 | 38.4 |

Example 7

Using each of liquid extract from dark standing cells of N-774 strain prepared as in Example 1 and said extract which has been irradiated with light as in Example 2, reaction solutions were prepared in accordance with the formulation shown in Table 7. The reaction was conducted at 0°C for a predetermined period of time at a dark place. A reaction velocity for every substrate shown in Table 7 was measured by measuring the amount of the starting nitrile consumed or that of the corresponding amide produced, using gas chromatography or high performance liquid chromatography. The ratio of the reaction velocity for the extract irradiated with light to that for the extract not irradiated is shown in Table 7.

Table 7

| Starting nitrite (g) | Liquid extract (ml) | 0.05M phosphate buffer (ml) | pH | Reaction velocity for light-irradiated extract |
|---|---|---|---|---|
| Acetonitrile (0.1) | 0.2 | 9.8 | 7.7 | 60.1 |
| Methacrylonitrile (0.1) | " | " | " | 61.0 |
| n-Valeronitrile (0.025) | " | " | " | 57.3 |
| Cyanopyridine (0.1) | " | " | " | 48.2 |
| Benzonitrile (0.0125) | " | " | " | 60.2 |
| Propionitrile (0.1) | " | " | " | 45.8 |
| n-butyronitrile (0.1) | " | " | " | 46.7 |
| Malononitrile (0.1) | " | " | " | 51.6 |
| Succinonitrile (0.1) | " | " | " | 47.3 |
| Fumaronitrile (0.1) | " | " | " | 41.1 |
| Chloroacetonitrile (0.1) | " | " | " | 48.3 |
| β-hydroxypropionitrile (0.1) | " | " | " | 53.1 |
| β-aminopropionitrile (0.1) | " | " | " | 52.9 |

## Claims

1. A process for hydrating a nitrile compound by the action of nitrilase produced by a microorganism to convert the nitrile compound into the corresponding amide compound,

11

EP 0 188 252 B1

characterized by
conducting the reaction under the conditions wherein:
(a) the nitrilase produced by the microorganism is in the form of crushed cells of the microorganism or intra-cellular substance obtained from the microorganism;
(b) the nitrilase produced by the microorganism is allowed to accept light energy of light having a wavelength of 100 to 1000 nm corresponding to at least $10\mu E/g$ microbial cells ($\mu$mol of photons/g microbial cells) before termination of the hydration reaction; wherein it is excluded that the accepted light energy is supplied solely by natural daylight; and
(c) the hydration reaction is carried out in a vessel composed at least partly of a material which does not permit transmission therethrough of light.

2. The process according to claim 1, in which the light energy of light having a wavelength of 100 to 1000 nm corresponds to at least about $20\mu E/g$ microbial cells.

3. The process according to any of Claims 1 to 2, in which the irradiation with light is carried out before and/or after contacting the nitrilase produced by the microorganism with the nitrile compound.

4. The process according to any of Claims 1 to 3, in which the microorganism belongs to the genus selected from the group consisting of Corynebacterium, Nocardia, Bacillus, Bacteridium, Micrococcus, and Brevibacterium.

5. The process according to any of Claims 1 to 4, in which the nitrile compound is selected from the group consisting of acetonitrile, propionitrile, n-butyronitrile, isobutyronitrile, n-valeronitrile, acrylonitrile, methacrylonitrile, benzonitrile, cyanopyridine, malononitrile, succinonitrile, fumaronitrile, chloroacetonitrile, $\beta$-hydroxypropionitrile, aminoacetonitrile, and $\beta$-aminopropionitrile.

**Patentansprüche**

1. Verfahrenen zur Hydratisierung einer Nitrilverbindung durch Einwirkung von Nitrilase, die von einem Mikroorganismus hergestellt worden ist, um die Nitrilverbindung in die entsprechende Amidverbindung umzuwandeln,
**dadurch gekennzeichnet,**
daß man die Umsetzung unter Bedingungen durchführt, unter denen
(a) die von dem Mikroorganismus erzeugte Nitrilase in Form von zerstoßenen Zellen des Mikroorganismus oder in Form von intracellulärer Substanz, die von dem Mikroorganismus erhalten ist, vorliegt;
(b) aur die von dem Mikroorganismus erzeugte Nitrilase Lichtenergie von Licht einwirken gelassen wird, das eine Wellenlänge von 100 bis 1000 nm entsprechend mindestens 10 $\mu E/g$ Microbenzellen ($\mu$mol Photonen/g Microbenzellen), bevor die Hydratisierungsreaktion beendet ist; wobei ausgeschlossen ist, daß die aufgenommene Lichtenergie allein von natürlichem Tageslicht ausgeht; und
(c) die Hydratisierungsreaktion in einem Gefäß durchgeführt wird, das zumindestens teilweise aus einem lichtundurchlässigen Material besteht.

2. Verfahren gemäß Anspruch 1, worin die Lichtenergie des Lichts mit einer Wellenlänge von 100 bis 1000 nm mindestens etwa 20 $\mu E/g$ Microbenzellen entspricht.

3. Verfahren gemäß einem der Ansprüche 1 oder 2, worin die Bestrahlung mit Licht vor und bzw. oder nach Inberührungbringen der von dem Mikroorganismus hergestellen Nitrilase mit der Nitrilverbindung durchgeführt wird.

4. Verfahren gemäß einem der Ansprüche 1 bis 3, worin der Mikroorganismus einem Genus aus der Gruppe Corynebacterium, Nocardia, Bacillus, Bacteridium, Micrococcus und Brevibacterium angehört.

5. Verfahren gemäß einem der Ansprüche 1 bis 4, worin die Nitrilverbindung aus der Gruppe Acetonitril, Propionitril, n-Butyronitril, Isobutyronitril, n-Valeronitril, Acrylnitril, Methacrylnitril, Benzonitril, Cyanopyridin, Malononitrile, Succinonitril, Fumaronitril, Chloracetonitril, $\beta$-Hydroxypropionitril, Aminoacetonitril, und $\beta$-Aminopropionitril ausgewählt ist.

**Revendications**

1. Procédé pour hydrater un nitrile par l'action d'une nitrilase produite par un microorganisme pour convertir le nitrile en l'amide correspondant,

   caractérisé en ce que l'on conduit la réaction dans les conditions dans lesquelles :

   (a) la nitrilase produite par le microorganisme est sous la forme de cellules broyées du microorganisme ou d'une substance intracellulaire obtenue à partir du microorganisme ;

   (b) la nitrilase produite par le microorganisme est admise à recevoir une énergie lumineuse de lumière ayant une longueur d'onde de 100 à 1000 nm correspondant au moins à 10 $\mu$E/g de cellules microbiennes ($\mu$mol de photons/g de cellules microbiennes) avant la fin de la réaction d'hydratation ; étant exclu que l'énergie lumineuse reçue soit fournie uniquement par la lumière du jour naturelle ; et

   (c) la réaction d'hydratation est conduite dans un récipient constitué au moins en partie d'une matière qui ne permet pas le passage de la lumière.

2. Procédé selon la revendication 1, dans lequel l'énergie lumineuse de lumière ayant une longueur d'onde de 100 à 1000 nm correspond au moins à environ 20 $\mu$E/g de cellules microbiennes.

3. Procédé selon l'une quelconque des revendications 1 et 2, dans lequel l'irradiation par la lumière est effectuée avant et/ou après la mise en contact de la nitrilase produite par le microorganisme avec le nitrile.

4. Procédé selon l'une quelconque des revendications 1 à 3, dans lequel le microorganisme appartient à un genre choisi dans le groupe formé par *Corynebacterium*, *Nocardia*, *Bacillus*, *Bacteridium*, *Micrococcus* et *Brevibacterium*.

5. Procédé selon l'une quelconque des revendications 1 à 4, dans lequel le nitrile est choisi dans le groupe formé par l'acétonitrile, le propionitrile, le *n*-butyronitrile, l'isobutyronitrile, le *n*-valéronitrile, l'acrylonitrile, le méthacrylonitrile, le benzonitrile, la cyanopyridine, le malononitrile, le succinonitrile, le fumaronitrile, le chloracétonitrile, le $\beta$-hydroxypropionitrile, l'aminoacétonitrile et le $\beta$-aminopropionitrile.